# EUROPEAN PATENT SPECIFICATION

(11) **EP 3 472 199 B1**
(45) Date of publication and mention of the grant of the patent: **01.11.2023**
(21) Application number: 17735015.4
(22) Date of filing: 15.06.2017
(51) Int. Cl.: C07K 16/18, G01N 33/68

(54) **COMP- PEPTIDE AND ANTIBODIES THERETO FOR DIAGNOSING OSTEOARTHRITIS**
COMP-PEPTID UND ANTIKÖRPER DAFÜR ZUR DIAGNOSE VON OSTEOARTHRITIS
COMP-PEPTIDE ET ANTICORPS DIRIGÉS CONTRE CELUI-CI POUR LE DIAGNOSTIC DE L'OSTÉOARTHRITE

(30) Priority: 16.06.2016 SE 1650855
(43) Date of publication of application: 24.04.2019
(73) Proprietor: SGPTH Life Science AB, 741 93 Knivsta (SE)
(72) Inventor: SKIÖLDEBRAND, Eva, 412 67 Göteborg (SE); LINDAHL, Anders, 436 40 Askim (SE); EKMAN, Stina, 741 93 Knivsta (SE)
(74) Representative: Noréns Patentbyrå AB
(86) International application number: PCT/EP2017/064667
(87) International publication number: WO 2017/216289

(56) References cited:
- WO-A1-2016/172722
- US-A1- 2004 214 272
- Anonymous: "COMP / THBS5 Antibody, Rabbit PAb, Antigen Affinity Purified Catalog Number: 10173-RP03", Sino Biological Inc., 6 March 2015 (2015-03-06), XP055404440, Retrieved from the Internet: URL:http://www.sinobiologicalcdn.com/reage nt/10173-RP03.pdf [retrieved on 2017-09-06]
- E. SKIÖLDEBRAND ET AL: "Cartilage oligomeric matrix protein neoepitope in the synovial fluid of horses with acute lameness: A new biomarker for the early stages of osteoarthritis", EQUINE VETERINARY JOURNAL., vol. 49, no. 5, 28 February 2017 (2017-02-28), pages 662-667, XP055404433, GB ISSN: 0425-1644, DOI: 10.1111/evj.12666
- Sally C Dickinson ET AL: "Cleavage of cartilage oligomeric matrix protein (thrombospondin-5) by matrix metalloproteinases and a disintegrin and metalloproteinase with thrombospondin motifs", Matrix Biology, 1 January 2003 (2003-01-01), pages 267-278, XP055404426, DOI: 10.1016/S0945-053XZ03.00034-9 Retrieved from the Internet: URL:http://www.sciencedirect.com/science/a rticle/pii/S0945053X03000349/pdfft?md5=605 7b01da47f60d10d0b7f7f406fb63f&pid=1-s2.0-S 0945053X03000349-main.pdf [retrieved on 2017-09-06]
- EUGENE Y. ZHEN ET AL: "Characterization of metalloprotease cleavage products of human articular cartilage", ARTHRITIS & RHEUMATISM, vol. 58, no. 8, 1 August 2008 (2008-08-01) , pages 2420-2431, XP055031747, ISSN: 0004-3591, DOI: 10.1002/art.23654

### SEQUENCE LISTING

<110> Skiöldebrand Eva
   Lindahl Anders
   Ekman Stina
<120> Peptide and antibodies for use in diagnosis
   <160> 6
<170> BiSSAP 1.3.6
<210> 1
<211> 5
<212> PRT
<213> Equus caballus
   <400> 1
   Ser Gly Pro Thr His
   1 5
<210> 2
<211> 9
<212> PRT
<213> Artificial Sequence
<220>
<223> Peptide used for immunisation
   <400> 2
   Ser Gly Pro Thr His Gly Gly Gly Cys
   1 5
<210> 3
<211> 9
<212> PRT
<213> Equus caballus
   <400> 3
   Ser Gly Pro Thr His Glu Gly Val Cys
   1 5
<210> 4
   <211> 15
<212> PRT
<213> Equus caballus
   <400> 4
   Pro Pro Gly Tyr Ser Gly Pro Thr His Glu Gly Val Gly Met Cys
   1 5 10 15
<210> 5
<211> 11
<212> PRT
<213> Equus caballus
   <400> 5
   Ser Gly Pro Thr His Glu Gly Val Gly Met Ala
   1 5 10
<210> 6
<211> 11
<212> PRT
<213> Homo sapiens
   <400> 6
   Ser Gly Pro Thr His Gln Gly Val Gly Leu Ala
   1 5 10

## Description

### FIELD OF THE INVENTION

This invention relates to a peptide and antibodies against this peptide, and their use in diagnosis, in particular diagnosis of systemic inflammatory disease, in particular osteoarthritis and cardiovascular disease.

### BACKGROUND

Atherosclerosis is associated with systemic low-grade chronic inflammation and the subsequent formation of inflammatory plaques in the blood vessels, and is one of the major causes of death in the western world. Atherosclerosis proceeds slowly from a less dangerous asymptomatic phase to the development of plaques to a phase where the plaques may cause thrombosis that may block capillaries thereby causing a heart attack or a stroke.

In the advanced phase, atherosclerosis frequently proceeds to carotid artery stenosis which is the narrowing of the inner surface of the carotid artery, caused by plaques. In this phase the risk of thrombosis formation and release of embolus causing stroke is very high.

It is today not possible to monitor how atherosclerosis proceeds to the dangerous phase.

Carotid stenosis is usually diagnosed by using ultrasound, which has moderate sensitivity and specificity. Therefore there is a need for methods to monitor the progression of atherosclerosis to carotid stenosis.

Osteoarthritis (OA) is a low-grade chronic systemic inflammatory that results from breakdown of joint cartilage and underlying bone. The disease proceeds from an early phase which often is characterized by inflammation and beginning disorganization of matrix proteins in the affected joints, to a more severe phase with damage to the underlying bone. The main symptom is pain in the joint. Osteoarthritis is a major clinical problem as it affects around 3.8% of the population. It is estimated that 50% of NSAID pain killer prescriptions are related to OA.

Although joint pain often leads a physician to suspect OA, especially if the patient is elderly, it is today difficult to diagnose the early stages of OA. Today diagnosis is often based on identification, using radiology, of irreversible structural damages typical of the late stages of OA. X-ray or MRI cannot, however, be used to diagnose early stage OA, because the structural damages are not yet visible. Also, X-ray and MRI examination requires expensive equipment, and making an appointment with a radiologist.

Osteoarthritis in horses is a great problem for horse owners. The principal reason for having a horse is to be able to use it, and often to compete with it. OA is the most frequent reason for not being able to use the horse and accounts for the greatest single economic loss in the horse industry.

Early OA in horses frequently manifests as lameness, i.e. an abnormal gait or stance. Lameness can often be subtle and it would be useful to have an improved method of monitoring lameness. This would enable horse owners to know when to rest horses from training and competing due to lameness.

OA in horses and humans are caused by similar mechanisms. In both humans and horses OA proceeds from an early stage characterized by inflammation over months and years to a later stage with extensive tissue damage (Goldring, M.B. and Otero M. Current Opinion in Rheumatology (2011) 23(5):471). OA disease mechanisms in humans and horses are also very similar on the molecular level (Stenberg J, Rüetschi U, Skiöldebrand E, Kärrholm J, Lindahl A. Proteome Sci. (2013) Oct 4;11(1):43) (Svala E, Löfgren M, Sihlbom C, Rüetschi U, Lindahl A, Ekman S, Skiöldebrand E. Connect Tissue Res. (2015);56(4):312-25.

Anonymous, 2015 discloses a polyclonal antiserum binding to the cartilage oligomeric matrix protein (COMP). WO 2016//172722) discloses 7-11 amino acid mer peptides used for the production of cancer immune therapy agents such as antibodies and diagnostic use. One of the neoepitopes is a peptide with sequence SGPTHASGN. US 2004/214272) discloses a 179 amino acid long plant protein with an N-terminal SGPTH sequence (see SEQ ID NO:309.498).

The document "COMP/THBS5 Antibody, Rabbit PAb, Antigen Affinity Purified Catalog Number: 10173-RP03", Sino Biological Inc, 6 March 2015, discloses an antibody against the protein COMP, without specifying the epitope.

Skioldebrand et al. "Cartilage oligomeric matrix protein neoepitope in the synovial fluid of horses with acute lameness: A new biomarker for the early stages of osteoarthritis" EQUINE VETERINARY JOURNAL, vol. 49, no. 5, 28 February 2017, is published after the priority date.

Dickinson et al: "Cleavage of cartilage oligomeric matrix protein (thrombospondin-5) by matrix metalloproteinases and a disintegrin and metalloproteinase with thrombospondin motifs", Matrix Biology, 1 January 2003 (2003-01 -01), pages 267-278, discusses the relevance of COMP in osteoarthritis.

Zhen et al: "Characterization of metalloprotease cleavage products of human articular cartilage", ARTHRITIS & RHEUMATISM, vol. 58, no. 8, 1 August 2008, pages 2420-2431, discloses various cleavage fragments of proteins.

WO 2016172722, which is filed before the priority date but published after the priority date, discloses various peptides.

US2004/214272 discloses various protein sequences.

The lack of early markers for OA hampers development of drugs that could be used to control the disease before it manifests as irreversible tissue damage. It would be useful if there were more convenient ways of diagnosing and staging OA.

This invention solves these and other problems.

### SUMMARY OF THE INVENTION

In a first aspect of the invention there is provided a method of diagnosis comprising, in a sample that previously has been isolated from a subject, analysing the sample for presence or amount of a peptide comprising the amino acid sequence N-terminal-SGPTH, where the serine residue has the NH₂ group of said peptide.

The method may be used for diagnosis of osteoarthritis or atherosclerosis, in particular early phase osteoarthritis.

In a second aspect of the invention there is provided an antibody that specifically binds to a peptide comprising the amino acid sequence N-terminal-SGPTH where the serine residue has the NH₂ group of said peptide, where the antibody binds to the sequence N-terminal SGTPH, for use in in vivo diagnosis. The diagnosis may be diagnosis of osteoarthritis or atherosclerosis in a subject. The atherosclerosis may be carotid artery stenosis. The antibody may be used for detecting the amount of a peptide comprising the amino acid sequence N-terminal-SGPTH where the serine residue has the NH₂ group of said peptide.

### BRIEF DESCRIPTION OF DRAWINGS

Fig. 1 The specificity of the anti-NH₂-SGPTH antibody was tested using a serial dilution of control peptide (SEQ ID NO 4) or SGPTHEGVC peptide (SEQ ID NO 3).
Fig. 2 is a graph that shows the concentration of peptide comprising the sequence NH₂-terminal SGPTH in synovial fluid samples from horses (*=p<0.050, ****=p<0.0001).
Fig. 3 is a graph that shows the concentration of peptide comprising the sequence NH₂-terminal-SGPTH in serum from humans (***=p<0.001).
Figs. 4 and 5 are images of samples of articular cartilage from horses, stained with the antibody against the NH₂-SGPTH peptide. The immunohistochemistry-stained sections are articular cartilage from a healthy horse (Healthy cartilage - figure 4) and a horse with superficial fibrillation of the articular cartilage (Early OA - figure 5). The antibody against the peptide stains negative in healthy cartilage. However, in early OA, the antibody stained positive (indicated by arrows) in the fibrillated area.
Fig. 6 shows a histological section of a sample of atherosclerotic plaque from a human patient, stained with the antibody against the NH₂-SGPTH peptide.

### DETAILED DESCRIPTION

Disclosed herein is a peptide comprising the sequence N-terminal-SGPTH (SEQ ID NO 1) where the serine residue has the NH₂ group of said peptide, an antibody against this peptide and the use of such an antibody in diagnosis.

The peptide may have a length of from 5 to 100, preferably from 5 to 30, more preferably from 5 to 20 amino acids, more preferably from 5 to 9 amino acids, as long as the N-terminal has the sequence SGPTH. The serine residue of this sequence of the peptide thus has the NH₂ group of the peptide.

In one disclosure the peptide has a length such that it can be used for immunization. The length of the peptide is then preferably at least nine residues.

The peptide may be an isolated peptide. The peptide may be isolated from for example synovial fluid, blood, plasma or serum. The peptide may also be synthesized, using methods known in the art, for example R. B. Merrifield (1963). "Solid Phase Peptide Synthesis. I. The Synthesis of a Tetrapeptide". J. Am. Chem. Soc. 85 (14): 2149-2154 and Schnolzer, M. A., P.; Jones, A.; Alewood, D.; Kent, S.B.H. (2007). "In Situ Neutralization in Boc-chemistry Solid Phase Peptide Synthesis". Int. J. Peptide Res. Therap. 13 (1-2): 31-44*.* The peptide may be an isolated peptide.

The peptide can be used for the production and isolation of an antibody. The antibody specifically binds to a peptide comprising the amino acid sequence N-terminal-SGPTH where the serine residue has the NH₂ group of said peptide, in particular a peptide that consist of at least nine amino acid residues. The term "antibody" also includes Fab, Fab', F(ab')2, Fv, and single-chain antibodies. Methods for producing antibodies against a peptide are well known. The peptide can be used for screening for antibodies that bind to the peptide and also for purifying the antibody.

Preferably the antibody has a high affinity for the peptide. Affinity can be expressed using the dissociation constant or Kd. Preferred binding affinities include those with a dissociation constant (Kd) less than 10⁻⁶ M, more preferably 5×10⁻⁷ M, more preferably 10⁻⁷ M, more preferably 5×10⁻⁸ M, 10⁻⁸ M, more preferably 5×10⁻⁹ M, more preferably 10⁻⁹ M, more preferably 5×10⁻¹⁰ M, more preferably 10⁻¹⁰ M, more preferably 5×10⁻¹¹ M, more preferably 10⁻¹¹ M, more preferably 5×10⁻¹² M, more preferably 10⁻¹² M, even more preferably 5×10⁻¹³ M, or and most preferably 10⁻¹³ M. Preferably the antibody is an isolated antibody. The antibody may be a purified antibody.

Preferably the antibody binds specifically to a peptide comprising the sequence N-terminal- SGPTH (SEQ ID NO 1) where the serine residue has the NH₂ group of said peptide. For generating the antibody it may be suitable to immunize with a peptide that is longer than SGPTH, for example the immunisation peptide SGPTHGGGC (SEQ ID NO 2). However, a subsequent screening step can identify antibodies that bind specifically to the epitope N-terminal-SGPTH where the serine residue has the NH₂ group of said peptide, and where the other residues of the peptide used for immunisation are not involved in antibody binding. In the immunisation peptide SGPTHGGGC (SEQ ID NO 2), the C-terminal cysteine may be used for conjugating the peptide, for example to a matrix to be used for antibody purification.

The antibody may be any antibody derived from a mammal such as mouse, rat, hamster, rabbit, goat, horse and the like, among which mouse is preferred or chicken. The isotype of the antibodies may be any of IgG, IgM, IgE, IgA, IgY and the like.

The antibody may be a polyclonal antibody, produced by immunisation of an animal, for example a rabbit, as is known in the art. But preferably the antibody is a monoclonal antibody. Preferably the monoclonal antibody is a mouse or rabbit monoclonal antibody. Monoclonal antibodies against the peptide may be generated using the well-known hybridoma technology (Kohler and Milstein, Nature, 256, 495-497, 1975). A single clone can be isolated by limiting dilution analysis, the soft agar assay, a method using a fluorescence activated cell sorter and the like. In the limiting dilution analysis, for example, colonies of the hybridoma are serially diluted to around 1 cell/well in a medium before cultivation to isolate the hybridoma which produces the desired antibody. The antibody may be a chimeric antibody or a humanized antibody.

Antibody clones may also be generated using other methods, such as, for example, phage display.

When the antibody is murine IgG, the antibody can be purified with affinity chromatography using a Protein A-conjugated carrier or an anti-mouse immunoglobulin-conjugated carrier.

There are well-known methods for producing, purifying and isolating antibodies and determining their binding capacity. It is referred to Current Protocols in Immunology and Current Protocols in Molecular Biology for details.

The antibody can be used for diagnosis in different manners. The antibody can be used for measuring the presence, the amount of or concentration of the peptide in a sample from a subject, which may be a human or an animal. The sample may be any type of biological sample, for example synovial fluid, plasma, serum, spinal fluid (liquor) or urine, ascites, or a histological section. Preferably the sample is a liquid sample. In a preferred embodiment the sample is a serum sample, a blood sample, a plasma sample or a sample of synovial fluid. In one even more preferred embodiment the sample is a sample of synovial fluid.

A convenient manner to measure the concentration of a peptide in a sample with an antibody is ELISA. The design and use of ELISA (enzyme-linked immunosorbent assay) is well known in the art of diagnostics. The antibody can also be used in, for example, immunohistochemistry. For example thin sections of tissue, for example tissue that is frozen, paraffin-treated or fixed, may be stained using the antibody as is known in the art of pathology. The antibody can also be used in western blot.

The antibody can be detected in various manners. A frequently used method is to use a secondary antibody that is conjugated with a substance that can be detected (a marker or label), for example an enzyme (such as HRP), a fluorophore or a radiolabel. For example, if the primary antibody is a mouse antibody, the secondary antibody can be a goat anti-mouse antibody. The presence of the marker can be detected with methods known in the art: an enzyme may be detected with reagents that produces a colour or light, a radiolabel may be detected with a scintillator or photographic film, and a fluorophore may be detected with a fluorescence detector or viewed in a fluorescence microscope.

Alternatively, the primary antibody (anti-SGPTH -antibody) may be directly conjugated with a marker/label.

A suitable working concentration of the antibody when it is used in various procedures such as ELISA or immunohistochemistry depends on the affinity of the antibody and can be determined by testing different concentrations of the antibody in order to find a concentration that gives a good signal to noise ratio. As an example, an antibody stock with a concentration of 1 mg/ml may be diluted at 1/100, 1/200, 1/1000 and 1/5000 for testing a suitable working concentration. Working concentrations of the antibody in these procedures is usually in the range of µg/ml, for example 1-10 µg/ml. The antibody is suitable diluted in PBS, possibly with the use of an additional protein such as BSA and a preservative, such as sodium azide.

The antibody can be used for diagnosis of a disease in a subject, in particular in a human or in a horse. For example the concentration of the peptide in a sample can be determined using standard methods, for example ELISA. The thus determined concentration can be compared against a standard value. A deviation from the standard value may be indicative of a particular disease, or a stage of the disease.

The diagnosis may be diagnosis of a disease associated with systemic inflammation in particular a systemic low-grade chronic inflammation. The diagnosis may thus be diagnosis of a neurodegenerative disease such as Alzheimer's disease, vascular dementia, amyotrophic lateral sclerosis (ALS), or a neuro- inflammatory disease such as multiple sclerosis, Parkinson's disease, Guillain-Barré syndrome, myastenia gravis, neuromyelitis optica, autoimmune encephalitis or narcolepsy.

In a preferred embodiment the disease being diagnosed may be one selected from the group consisting of atherosclerosis and carotid artery stenosis, in a particular in a human. In particular the disease can be carotid artery stenosis. The sample is then preferably a blood sample, a plasma sample or a serum sample.

The antibody can be used for monitoring the advance of atherosclerosis in humans, in particular from plaque disease to carotid artery stenosis. The antibody may be used for distinguishing patients with plaques only from patients with carotid artery stenosis. A peptide concentration, based on the current calibrator with the rabbit polyclonal antisera, of above 100 ng/ml, more preferably above 600 ng/ml, more preferably above 800 ng/ml, more preferably 1000 ng/ml more preferably 1200 ng/ml and most preferably above 2000 ng/ml in serum may be indicative of carotid artery stenosis in a human. The appropriate cut-off may be established using standard experiments and appropriate controls.

The antibody can be used for diagnosis of OA in humans or in horses. In a preferred embodiment, high concentration of the peptide in synovial fluid may indicate early OA. Low concentration of the peptide may indicate no OA or late stage of OA. The antibody can be used for monitoring the proceeding from acute lameness/early OA to chronic lameness/chronic OA, in particular in horses. In horses, the antibody may be used to diagnose one condition selected from the group consisting of acute lameness/early OA from and chronic lameness /chronic OA.

In the horse, a peptide concentration in synovial fluid concentration, based on the current calibrator with the rabbit polyclonal antisera, of above 1 µg/ml, more preferably over 20 µg/ml, more preferably 30 µg/ml more preferably above 40 µg/ml more preferably over 200 µg/ml may be indicative of early lameness and thus early OA. The cut-off levels for horses may be established using standard experiments and appropriate controls.

The antibody and the necessary reagents may be included in a kit for detecting the peptide in a sample. The kit may be based on ELISA, for example competitive ELISA. The kit may include a stationary phase (such as a plate with wells), secondary antibodies, buffers and reagents for detecting the marker.

### EXAMPLE 1

### Polyclonal antibody

A rabbit polyclonal antibody was raised against the immunisation peptide N-terminal SGPTHGGGC-C-terminal, SEQ ID NO 2) where the serine residue had the NH₂ group of said peptide. The peptide n-terminal SGPTH was identified as a cleavage fragment of the COMP protein (thrombospondin 5) in synovial fluid in horses. The human protein has the same sequence. The immunogenic peptide was used for antibody purification from rabbit serum. The polyclonal antibody was used in examples 2-6.

### EXAMPLE 2

Inhibition ELISA, detection of the peptide in the horse and human.

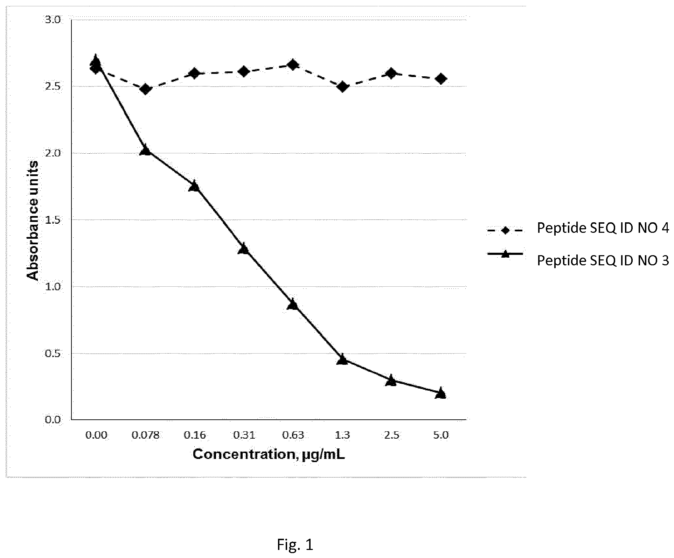

An inhibition ELISA was developed for a peptide with the sequence SGPTHEGVC (SEQ ID NO 3) by using the antibody from Example 1. The ELISA was carried out as in Example 3 (Horse) and Example 4 (Human). The binding of the antibody to this peptide confirm that the polyclonal antibody is specific for N-SGPTH. Also the antibody did not bind to the uncleaved control peptide with the sequence PPGYSGPTHEGVGMC (SEQ ID NO 4), showing that the N-terminal is a part of the epitope for this antibody, since this peptide (SEQ ID NO 4) comprises SGPTH but not the N-terminal of N-SGPTH (Fig 1). As expected, in inhibition ELISA the SGPTHEGVC (SEQ ID NO 3) peptide gives a decreasing absorbance signal with increasing peptide concentration. ELISA was validated in serum and synovial fluid (SF) from both human and horse. Thus it was established that the antibody detects peptide present in blood and synovial fluid from human and horse.

The intra assay validation coefficient of variation (CV) (%) of 10.9 and an inter assay validation CV (%) of 10.7 (the lowest limit of quantification was 0.156 µg/ml) for the horse assay. The CV for human serum samples was similar to the horse data. The serum and synovial samples have also been tested regarding the stability after freezing (-80°C), thawing and when adding protease inhibitors as well as EDTA.

### EXAMPLE 3

### Detection of the peptide in synovial fluid from horses with osteoarthritis

Synovial fluid (SF) samples were collected from biobanks. The material used comprises synovial fluid from the middle carpal and fetlock joints from horses.

The inclusion criteria for the present samples were A) more than 100 µl of available fluid, and one of the following conditions: B) a history of early OA/acute lameness (<4 weeks duration), C) a history of chronic OA/chronic lameness (>4 weeks duration), D) a history of no lameness and a joint with normal morphology (healthy control), or E) a joint with a late stage OA present.

Biobank I included 28 young Standard-bred (STB) trotters (13 fillies and 15 colts) trained by the same trainer at the same campus. These horses started their training at a mean age of 19.5 months and finished at a mean age of 40 months. All of the horses were clinically healthy and were examined on six occasions (visits 1, 2, 3, 4, 5 and 6). At the SF sampling, a clinical lameness examination with a flexion test including radiographs and scintigraphy of the carpal joints was performed.

Biobank II consisted of left carpal joints from STB and Swedish warm-blooded riding horses (SWHs) that were euthanized at one abattoir. The SF from the left intercarpal joint was sampled immediately post mortem. The articular cartilage was characterized as normal or with structural OA lesions of the proximal articular surface at the third carpal bone.

Biobank III consisted of SF from clinically lame horses. The horses were examined during a routine lameness examination including flexion tests and intra-articular anaesthesia. This examination also included the lameness locator test to evaluate lameness before and after anaesthesia. The SF was collected from different joints depending on the clinical lameness evaluation. This bank consists of SF from joints with defined lameness in a specific joint via the intra articular anaesthesia and lameness locator.

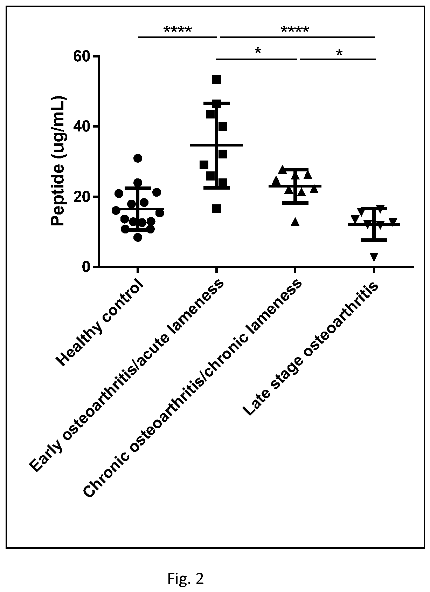

The material used in figure 2 is SF from the middle carpal and fetlock joints from horses with macroscopically normal articular cartilage, healthy controls (n=8, Biobank II), mild-to-moderate structural OA changes of the articular cartilage, late stage OA (n=8, Biobank II), early OA/acute lameness <4 weeks (n=9, Biobank III) and chronic OA/chronic lameness >4 months (n= 9, Biobank III) from young healthy racehorses in training, healthy controls (n=7, Biobank I).

An inhibition ELISA was developed to quantify the concentration of the peptide in SF. NUNC plates were coated with 4.0 µg/mL peptide (sequence SGPTHEGVC, SEQ ID NO 3) diluted in 0.1 M carbonate buffer, pH 9.6 and incubated at 4°C overnight. A serial dilution of 5.0 µg/mL peptide (sequence SGPTHEGVGMA (SEQ ID NO 5) in 10 mM PBS with 0.6% BSA and 0.8% SDS was used as a calibration curve (range=5-0.078 ug/mL). SF samples were diluted 1:20 in PBS with 0.84% SDS. Duplicates of standard and SF were incubated in 96-well Sterilin plates at 25°C overnight. On the second day, primary anti-SGPTH antibody (diluted 1:2000 in PBS with 1 % BSA and 4 % Triton-X-100) was added to the Sterilin plates and the plates were incubated for 1 h 20 min at 25 °C. NUNC plates were washed and blocked with PBS with 1% BSA and 0.1% Tween for 1 h, at 25°C. A total of 100 µL was transferred from the Sterilin plates to the NUNC plates and incubated for 1 h at 25°C. After incubation, the NUNC plates were washed, and secondary antibody (Goat Anti-Rabbit IgG H&L [HRP]), diluted 1:20,000 in PBS with 1% BSA and 0.1% Tween was added. The plates were incubated for 1 h at 25°C and then washed six times and incubated with substrate (Substrate Reagent Pack DY999, R&D System, UK) for approximately 8 min at 25°C. Stop solution (1 M H₂SO₄) was added, and the absorbance was measured at 450 nm (SpectraMaxPlus 384, MDS Analytical Technologies Ltd, UK).

There was a statistically increased concentration of peptide in synovial fluid from horses with early osteoarthritis/acute lameness compared to healthy control joints and joints from horses with chronic osteoarthritis /chronic lameness and/or having late stage osteoarthritis (Fig 2).

Data are presented as mean ± SD and differences between means were assessed by one-way ANOVA using GraphPad Prism 6 (GraphPad Software Inc, San Diego, CA, USA). For comparisons between the groups a Turkey's multiple comparisons test was performed. The level of significance was set at p<0.05.

### EXAMPLE 4

Detection of the peptide in serum from patients with atherosclerosis. Patient material was as in Strömberg S, Nordanstig A, Bentzel T, Österberg K, Bergström GM, Risk of early recurrent stroke in symptomatic carotid stenosis, Eur J Vasc Endovasc Surg (2015); 49: 137-44.

An inhibition ELISA was developed to quantify the concentration of the peptide in serum. NUNC plates were coated with 4.0 µg/mL peptide (sequence SGPTHEGVC (SEQ ID NO 3) diluted in 0.1 M carbonate buffer, pH 9.6 and incubated at 4°C overnight. A serial dilution of 1000 ng/mL to 15,6 ng/ml peptide (sequence SGPTHQGVGLA (SEQ ID NO 6) in 10 mM PBS with 0.6% BSA and 0.8% SDS was used as a calibration curve. Serum samples were diluted 1:4 in PBS with 0.84% SDS. Duplicates of standard and serum were incubated in 96-well Sterilin plates at 25°C overnight. On the second day, primary anti-SGPTH antibody (diluted 1:6000 in PBS with 1 % BSA and 4 % Triton-X-100) was added to the Sterilin plates and the plates were incubated for 1 h 20 min at 25 °C NUNC plates were washed and blocked with PBS with 1% BSA and 0.1% Tween for 1 h, at 25°C. A total of 100 µL was transferred from the Sterilin plates to the NUNC plates and incubated for 1 h at 25°C. After incubation, the NUNC plates were washed, and secondary antibody (Goat Anti-Rabbit IgG H&L-HRP), diluted 1:20,000 in PBS with 1% BSA and 0.1% Tween was added. The plates were incubated for 1 h at 25°C and then washed six times and incubated with substrate (Substrate Reagent Pack DY999, R&D System, UK) for approximately 21 min at 25°C. Stop solution (1 M H₂SO₄) was added, and the absorbance was measured at 450 nm (SpectraMaxPlus 384, MDS Analytical Technologies Ltd, UK).

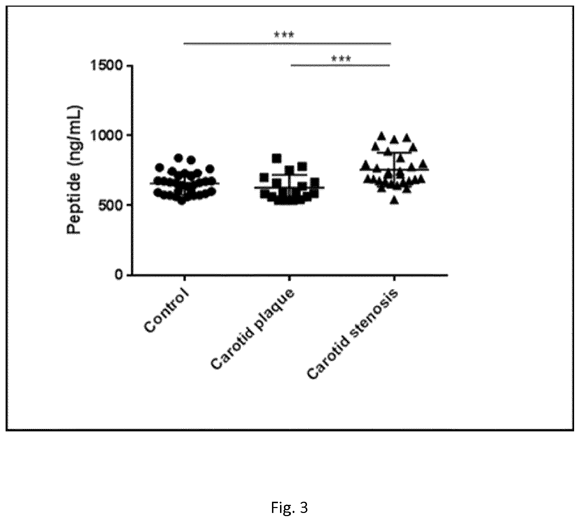

There was a statistically increased concentration of peptide in serum from patients with carotid plaques compared to patients with carotid stenosis (Fig 3).

Statistics were as in Example 3.

### EXAMPLE 5

### Immunohistochemistry of articular cartilage form horses

Formalin-fixed blocks of healthy and early OA articular cartilage, were immuno-stained for the peptide (rabbit polyclonal antibody). The polyclonal antibodies were used at a dilution of 1:800 and 1:1000 respectively. In brief, specimens were sectioned and mounted onto slides, deparaffinized, rehydrated and washed in phosphate-buffered saline (PBS; 0.01 M, pH 7.4. Endogenous peroxidase activity was quenched with 3 % hydrogen peroxide in PBS. Non-specific binding was blocked by incubating the sections in 2% normal goat serum (DAKO, X0907, rabbit antibody) then dried, and incubated with the antibody for 60 min. at room temperature (RT). After rinsing in PBS, the sections were incubated with horseradish peroxidase (HRP)-conjugated secondary antibodies (Dako Real EnVision^{™} Detection System, k5007, ready-to-use kit) at 30 min. in RT. Visualization was performed using the color developer 3, 3-diaminobenzidine (DAB+ Cromogen Dako EnVision) and counterstaining with haematoxylin. As negative controls, the primary antibody was substituted with nonimmune-rabbit-serum (Rabbit Immunoglobulin Fraction X0936, DAKO) in the same dilution as the primary antibody.

The sections were evaluated subjectively using Nikon Eclipse E600 microscope and NIS Elements Basic Research software version 3.22.11 (Nikon Instruments Inc, Melville, NY, USA).

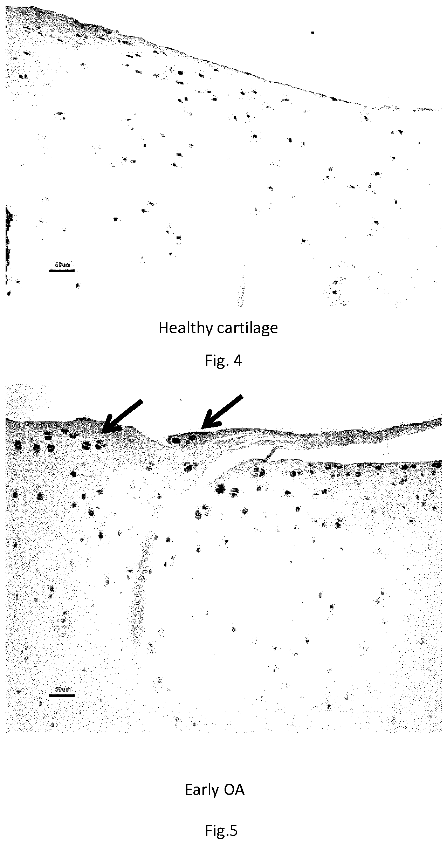

Immunohistochemistry slides of articular cartilage from a healthy horse (healthy cartilage, Fig 4) and horse with superficial fibrillation (a damage on the surface of the cartilage) of the articular cartilage (early OA) shows distinct staining patterns (Fig 5). The antibody against the peptide stains only weakly or negative in healthy cartilage. However, in early OA, the antibody stains positive in the fibrillated area.

### EXAMPLE 6

Immunohistochemistry of atherosclerotic plaques from human from a material described in Fagerberg B, Ryndel M, Kjelldahl J, et al. J Vase Res (2010); 47: 221-30.

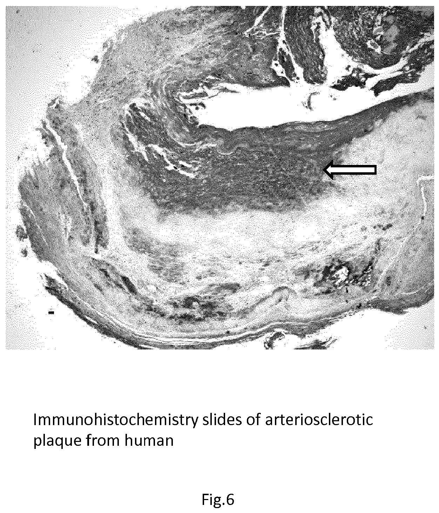

A sample from a patient with symptomatic carotid stenosis was examined with magnetic resonance angiography to localize the maximum stenosis in-vivo, prior to endarterectomy. Transverse tissue sections of the maximum stenosis were used for immunohistochemistry, which was carried out as in Example 5. There was increased staining (indicated by arrow) in the intima wall, indicating prescence of peptide comprising the sequence N-SGPTH (Figure 6).

This example 6 and example 4 shows that the antibody can be used for diagnosis of atherosclerosis. Furthermore, example 3 and example 5 shows that the antibody can be used for diagnosis of osteoarthritis. The common denominator for osteoarthritis and atherosclerosis is systemic inflammation. We conclude that the antibody can be used for diagnosis of disease where systemic inflammation is present, such as for example osteoarthritis, atherosclerosis, and neurodegenerative disorders such as Alzheimer's disease and vascular dementia and neuro-inflammatory diseases such as multiple sclerosis.

## Claims

1. A method of diagnosis comprising: analysing a sample that previously has been isolated from a subject for presence or amount of a peptide comprising the amino acid sequence N-terminal-SGPTH where the serine residue has the NH₂ group of said peptide

2. The method of claim 1 where the method is used in the diagnosis of osteoarthritis or atherosclerosis.

3. The method of claim 2 where the osteoarthritis is early phase osteoarthritis.

4. An antibody that specifically binds to a peptide comprising the amino acid sequence N-terminal-SGPTH where the serine residue has the NH₂ group of said peptide, where the antibody binds to the sequence N-terminal SGTPH, for use in in vivo diagnosis.

5. The antibody for use according to claim 4 where the diagnosis is diagnosis of osteoarthritis or atherosclerosis in a subject.

6. The antibody for use according to claim 5 where the diagnosis is diagnosis of atherosclerosis.

7. The antibody for use according to claim 6 where the atherosclerosis is carotid artery stenosis.

8. The antibody for use according to any one of claims 4 to 7 where the antibody is used for detecting the amount of a peptide comprising the amino acid sequence N-terminal-SGPTH where the serine residue has the NH₂ group of said peptide.

## Patentansprüche

1. Diagnoseverfahren, umfassend:
Analysieren einer Probe, die zuvor aus einem Subjekt isoliert wurde, hinsichtlich eines Vorhandenseins oder einer Menge eines Peptids, das die Amino-Säuren-Sequenz N-terminal-SGPTH umfasst, wobei der Serin-Rest die NH₂-Gruppe des Peptids aufweist.

2. Verfahren gemäß Anspruch 1, wobei das Verfahren zur Diagnose von Ostheoarthritis oder Atherosklerose verwendet wird.

3. Verfahren gemäß Anspruch 2, wobei es sich bei der Ostheoarthritis um eine Ostheoarthritis früher Phase handelt.

4. Antikörper der sich spezifisch an ein Peptid bindet, das die Amino-Säuren-Sequenz N-terminal-SGPTH umfasst, wobei der Serin-Rest die NH₂-Gruppe des Peptids aufweist, wobei sich der Antikörper an die Sequenz N-terminal-SGPTH bindet, zur Verwendung in einer In-vivo-Diagnose.

5. Antikörper zur Verwendung gemäß Anspruch 4, wobei es sich bei der Diagnose um eine Diagnose von Ostheoarthritis oder Atherosklerose bei einem Subjekt handelt.

6. Antikörper zur Verwendung gemäß Anspruch 5, wobei es sich bei der Diagnose um eine Diagnose von Atherosklerose handelt.

7. Antikörper zur Verwendung gemäß Anspruch 6, wobei es sich bei der Atherosklerose um eine Stenose der Arteria Carotis handelt.

8. Antikörper zur Verwendung gemäß einem der Ansprüche 4 bis 7, wobei der Antikörper zur Feststellung der Menge eines Peptids verwendet wird, das die Amino-Säuren-Sequenz N-terminal-SGPTH umfasst, wobei der Serin-Rest die NH₂-Gruppe des Peptids aufweist.

## Revendications

1. Méthode de diagnostic comprenant, l'analyse d'un échantillon qui a été au préalable isolé à partir d'un sujet pour la présence ou la quantité d'un peptide comprenant la séquence d'acides aminés extrémité-N-SGPTH dans l'échantillon, où le résidu de sérine a le groupe NH₂ dudit peptide.

2. Méthode selon la revendication 1, laquelle méthode est utilisée dans le diagnostic d'une arthrose ou d'une athérosclérose.

3. Méthode selon la revendication 2, dans laquelle l'arthrose est une arthrose à un stade précoce.

4. Anticorps qui se lie spécifiquement à un peptide comprenant la séquence d'acides aminés extrémité-N-SGPTH où le résidu de sérine a le groupe NH₂ dudit peptide, lequel anticorps se lie à la séquence extrémité-N-SGPTH, pour une utilisation dans un diagnostic in vivo.

5. Anticorps pour une utilisation selon la revendication 4, dans lequel le diagnostic est un diagnostic d'arthrose ou d'athérosclérose chez un sujet.

6. Anticorps pour une utilisation selon la revendication 5, dans lequel le diagnostic est un diagnostic d'athérosclérose.

7. Anticorps pour une utilisation selon la revendication 6, dans lequel l'athérosclérose est une sténose de l'artère carotide.

8. Anticorps pour une utilisation selon l'une quelconque des revendications 4 à 7, lequel anticorps est utilisé pour détecter la quantité d'un peptide comprenant la séquence d'acides aminés extrémité-N-SGPTH où le résidu de sérine a le groupe NH₂ dudit peptide.
